# EUROPEAN PATENT APPLICATION

(11) **EP 2 022 847 A1**
(43) Date of publication of application: **11.02.2009**
(21) Application number: 07015532.0
(22) Date of filing: 07.08.2007
(51) Int. Cl.: C12N 5/06

(54) **Pluripotent stem cells, methods for their isolation and their use and culture media**

(71) Applicant: Virant-Klun, Irma, 1000 Ljubljana (SI)
(72) Inventor: Virant-Klun, Irma, 1000 Ljubljana (SI)
(74) Representative: Leissler-Gerstl, Gabriele

(57) **Abstract**

Pluripotent stem cells can be obtained from follicular fluid. Methods for obtaining pluripotent stem cells and their use as well as culture media are described.

## Description

The present invention is concerned with pluripotent stem cells isolated from follicular fluid, a method for isolating stem cells and methods for differentiating the stem cells, and a culture medium that is useful for these methods.

The term stem cells is used for non-differentiated cells that are naturally present in different organs of the human body, which can proliferate as undifferentiated cells or can differentiate into different types of cells. Stem cells (SC) can be *totipotent, pluripotent, multipotent*, and *monopotent*. Totipotent SC give rise to both embryo and placenta. The totipotent stem cell is a fertilized oocyte (zygote) or first blastomeres after cleavage of zygote. Pluripotent SC give rise to all three germ layers of the embryo. Pluripotent SC are stem cells from the inner cell mass (ICM) of the blastocyst or epiblast stem cells (EPSC). They could be also derived as immortalized cell lines in *in vitro* cultures from i) ICM cells (ESC) or from ii) primordial germ cells (EG). Pluripotent SC can be differentiated to any type of cells *in vitro*. Multipotent SC give rise to one of the germ cell layers only, either ecto-, meso- or endoderm. Their ability to differentiate *in vitro* is limited. Monopotent SC are tissue-commited stem cells that give rise to cells of one lineage, for example, epidermal stem cells, hematopoietic stem cells, intestinal epithelium stem cells, neural stem cells, skeletal muscle stem cells, or liver stem cells.

The isolation of stem cells from embryos has raised a lot of moralic and ethical questions and embryos are a very limited source if any for isolating new stem cells.

A possible source for adult stem cells are human organs. However, it is very difficult to isolate stem cells from adult organs as they are very rare. Moreover, these adult stem cells are more restricted in their potency of differentiation into different cell types than embryonic stem cells.

Stem cells with embryonic stem cells character have been postulated and are desirable for the provision of oocytes that could be used for infertility treatment. The long time persisting dogma that the total number of follicles and oocytes are determined at birth and excluding ovarian stem cells was never completely confirmed. All the time it was strongly criticized. In 1870 and 1917 Waldeyer (1) and Kingery (2) postulated, that neo-oogenesis and folliculogenesis can occur in the adult ovary from the ovarian surface epithelium (OSE) stem cells with embryonic stem cells character, termed also as germinal epithelium. Kingery has confirmed de novo formation of oocytes from the surface epithelium of adult ovary in white mouse. American pioneers of modem reproductive physiology - Allen (3) and Evans (4) have concluded from their experimental work in the mouse, that new oocytes can be formed from OSE stem cells in adult period of life. In spite of all criticism the dogma has persisted until now.

Nowadays there is still a lot of theoretical criticism of the dogma (5, 6, 7). Also some experimental work show, that the persisting dogma might be wrong. In oncology it is confirmed, that agressive ovarian tumors are formed by uncontrolled proliferation of non-differentiated stem cells in the OSE cell layer (8). Johnson and his co-workers (9) from Tilly's group at Harvard University, USA, have destroyed all primordial follicles in pre-pubertal mices by the toxicant busulphan, and confirmed the presence of meiotic active oocytes in ovaries of the same animals in adult period of life. They supposed for oocyte-like cells to develop from stem cells. They also found oocyte-precursors - embryonic stem cells in the bone marrow of mouse (10). Wright and his co-workers (11) have found a high telomerase activity in the human fetal, newborn, and adult human OSE. Parrott and his co-workers (12) have found the expression of c-kit ligand/stem cell factor in human OSE. Bukovsky and his research team at University Tennessee, USA, scrapped human OSE from ovarian biopsates, set-up cell culture, and confirmed the presence of different cell types, including oocyte-like cells in the cell culture in postmenopausal women (13). This confirmed indirectly the possible presence of stem cells in human OSE.

In the prior art several approaches have been made to provide stem cells and to solve the above mentioned problems.

WO 2007/008377 discloses that adult ovarian stem cells can be isolated from ovarian surface epithelium and can differentiate to oocytes that can be fertilized and transferred into the uterus. The donor of the cells is the recipient of the fertilized oocyte and the method provided in this publication shall enable women with premature ovarian failure (with no naturally present oocytes and follicles) to become pregnant. However, the source for the cells used is not generally available and the OSE cells can be obtained by a surgical operation.

Moreover, US 6893871 B2 proposes a method for the cultivation of human ovarian mesothelial cells from fetusses. However, the use of embryos or fetusses is banned in many countries and raises a lot of ethical concerns.

Although is has been found possible to isolate granulosa cells from follicular fluid and to set-up successfully granulosa cell culture (14), and although it was tried to use follicular fluid for cultivation of oocytes (15), there was no successful cultivation of pluripotent cells from this source until now.

Therefore, extensive searches have been made to provide a source for stem cells without using embryos and without using surgical operation.

As on the one hand stem cells are deemed to be a very valuable source for research and for regenerative medicine and on the other hand the source for stem cells is very limited, it was the object of the present invention to provide a new source for stem cells. Moreover, it was an object of the present invention to provide methods to obtain and culture pluripotent stem cells. The use of stem cells is also an object of this invention.

According to the present invention follicular fluid is the source for pluripotent stem cells. Follicular fluid is used to isolate stem cells and to culture them.

It was surprisingly found by the present inventor, that it is possible to establish succesfully the cell culture from »waste« follicular fluid retrieved in the program of in vitro fertilization. In this cell culture different cell types differentiate, including oocyte-like cells. Furthermore it was found that it is possible to isolate from the follicular fluid a special subpopulation of cells that are positive for markers of embryonic stem cells, which differentiate spontaneously into different cell types. This type of cells has not been isolated until now.

The inventor surprisingly found that follicular fluid, which is only a - normally discarded - byproduct of in vitro fertilization program, is a very valuable source for the isolation of pluripotent stem cells with embryonic stem cell character. Moreover, it was found that follicular fluid is a valuable source for an additive for cultivation of and differentiation to different cell types from human stem cells isolated therefrom.

It was also surprisingly found that the use of follicular fluid serum as additive for culture medium improves the differentiation of stem cells to different cell types or progenitor cells respectively.

Thus, according to one aspect the present invention provides a method for obtaining pluripotent stem cells from follicular fluid.

In a further aspect, the present invention is concerned with pluripotent stem cells that are capable of differentiating in vitro into different cell types, such as oocytes, neurons, myoblasts or fibroblasts.

Another aspect of the present invention is culturing pluripotent stem cells obtained from follicular fluid for differentiation and recovering and isolating differentiated cells.

A further aspect of the present invention is a method for culturing pluripotent stem cells obtained from follicular fluid by growing the pluripotent stem cells in a culture medium that contains up to 30% by volume, preferably 5 to 25% by volume of follicular fluid serum, preferably follicular fluid serum obtained from the same follicles.

A further aspect of the present invention is the use of pluripotent stem cells obtained from follicular fluid for regenerative medicine.

An aspect of the present invention is also the use of pluripotent stem cells obtained from follicular fluid that differentiate to oocyte-like cells for in vitro fertilisation, particularly for women with ovarian infertility, especially for women with poor ovarian response to hormonal stimulation in in vitro fertilisation programs..

A further aspect of the present invention is the use of follicular fluid for preparing a medium for culturing stem cells in general or as differentiation medium for stem cells.

Thus, the present invention is based on two surprising findings, namely that follicular fluid can be a source for stem cells and that follicular fluid is an ingredient for culture mediums that enables cultivation and differentiation of stem cells..

Until now embryonic stem cells could be obtained only from human pre-implantation embryos, which provides problems. It is the gist of the present invention that it is now possible to prepare pluripotent stem cells from a fluid which normally is discarded.

Although there have been some attempts to isolate stem cells from human ovarian surface epithelium, it was not possible to yield viable mature oocytes for fertility treatment and other cell types by cultivating the cells in known culture media. It has now surprisingly been found that stem cells from follicular fluid as well as stem cells obtained from ovarian surface epithelium can be cultured successfully, Moreover, stem cells can differentiate into different cell types if follicular fluid serum is added to the culture medium. The methods and results will be presented below.

The invention is also illustrated by drawings wherein
Figure 1 is a photograph showing until now non-defined ovarian cells in the human ovary, responding to stem cells (left - the first type of round cells with a small bubble-like structure and right - the second type of round cells whit a bubble-like appearance) (inverted microscope, magnification X 400). At the left side down is an erythrocyte.
Figure 2 is a photograph showing a first type of stem cells with a small bubble-like structure that are also positive for c-kit (marker of early germinal cells) and and a second type of oocyte-like cell (big cell) after immunostaining (green colour; fluorescent microscope, magnification X 400).
Figure 3 is a photograph showing fibroblasts, developed in follicular fluid cell culture.
Figure 4 is a photograph showing neurons, developed in follicular fluid cell culture
Figure 5 is a photograph showing a myoblast, developed in follicular fluid cell culture.
Figures 6 a) to c) are photographs showing oocyte-like cells, developing in follicular fluid cell culture (attachment).

The follicular fluid that according to present invention is used as basic material and source for stem cells is available inter alia as remainder from in vitro fertilisation (IVF) where it otherwise is discarded. The follicular fluid is the source for isolating pluripotent stem cells of the present invention. Ovarian follicles are the basic unit for human reproduction. Each oocyte is contained in a follicle which for the primary follicle is only an epithelium layer but grows and develops in the ovary. The primary follicle consists of a primary oocyte with a single layer of cuboidal/columnar follicular cells. As development proceeds, the number of follicular cells increases by mitosis forming several layers around the primary oocyte. The primary follicle becomes a secondary follicle which is surrounded by the several layers of cuboidal/columnar follicular cells, now collectively called the membrana granulosa which begin to secrete follicular fluid. In the further development follicular fluid fills the inner part. In early tertiary follicles, granulosa cells grow which are enclosed in a thin layer of an extra-cellular matrix outside which theca interna and theca externa are found. During ovulation the Graafian follicle develops which is a mature vesicular follicle having a diameter of about 20 mm and has a relatively high fluid pressure. During ovulation the outer layer of the follicle ruptures and the oocyte is pushed out, ready to be fertilized by a sperm. The fluid in the follicle that can be aspirated or recovered from the follicle before or after expulsation of the oocyte is the basic material for the present invention.

For in vitro fertilization ovaries of women are stimulated by gonadotrophins to develop and mature as much oocytes as possible. In the ovary each oocyte develops embedded in the structure a follicle. It is possible to monitor by ultrasound the development of the follicles in the ovary and when the follicles are big and mature enough according to the diameter measured and according to the estradiol level measured in the blood, oocytes are aspirated from follicles. From each follicle follicular fluid can be aspirated for example by an ultrasound-guided needle. Together with the follicular fluid oocytes are removed to perform the in vitro fertilization. When oocytes are removed for in vitro fertilisation, follicular fluid can be used.

The follicular fluid remaining from this in vitro fertilization treatment is collected and can be used for the isolation of the stem cells. It is possible to provide autologous stem cells if follicular fluid is obtained from a woman and is used afterwards for preparing stem cells for this donor. It is also possible to use follicular fluid obtained from infertility treatment of patients for providing heterologous stem cells, i.e. for the treatment of other persons than the donor. The follicular fluid can be used as obtained or can be frozen and stored and then be thawed when stem cells should be isolated.

It is preferred to test the follicular fluid donor for infectious diseases like HIV, hepatitis c and a and cytomegalovirus to avoid contamination.

Preferably the work with the follicular fluid and the stem cells is carried out under sterile conditions and at a temperature of about 30 to 40°C, particularly at about 37°C.

To set up a cell culture the follicular fluid is centrifuged for a period sufficient to aggregate the cells. A period of 5 to 20 minutes, preferably 7 to 12 min is normally sufficient. The conditions are as normally used for recovering cells, i.e. about 1000 to 5000 rpm, preferably 2000 to 3000 rpm.

After centrifugation the pellet is recovered and is then resuspended in culture medium. The culture medium can be any medium that is known for cell culture such as DMEM/F-12 medium. An example of a culture medium for human ovarian surface epithelium (HOSE) cells is one consisting of Medium199:MCDB105 (1:1 v/v) supplemented with fetal calf serum (15% v/v), streptomycin (50 µg/ml), penicillin (50 IU/ml) and L-glutamine (2 mM) (Yong *et al*. 2002). A preferred medium is DMEM/F-12 medium. Additives like buffering agents, agents having estrogenic activity, antibiotics and other well known additives can be added to the culture medium in amounts that are usual for this purpose and are well-known to the skilled artisan. It is preferable to preincubate the culture medium in a CO₂-incubator for some time and only thereafter add the cell pellet obtained by centrifugation.

It has been found by the inventor of the present application that adding additionally heat-inactivated serum from follicular fluid to the cell suspension improves or sometimes even enables the cultivation of stem cells. Therefore, preferably a culture medium is used which contains up to 30, preferably 5 to 25%, based on the volume of the final culture medium, of heat-inactivated serum of follicular fluid. The serum can be of the same follicular fluid that has been used for isolating the cells or can be a serum of follicular fluid that has been obtained from another sample.

It has been found that serum obtained from follicular fluid is a very valuable additive for culture media and improves the cultivation of different types of cells. It contains different hormones at high levels, for example estrogen, progesterone, FSH, and androgens. The follicular fluid protein - albumin concentration is higher than the respective concentrations in blood plasma (2). The concentration of basic amino acids is almost twice as high in the follicular fluid as in blood plasma (2). Concentrations of Asp, Thr, Glu, Glu-NH2, Gly, Ala, and Met in follicular fluid is much higher than in blood plasma, and other biological fluids. In follicular fluid there is a high concentration of lipid substances - free cholesterol and follicular fluid meiosis-activating sterol (FF-MAS) (3, 8). Follicular fluid is also characterized by high concentrations of growth factors (epidermal growth factor, insulin-like growth factor-I, and inhibins A and B) (4). In follicular fluid high concentrations of proteoglycans (5), lipoproteins (6), estradiol fatty acid esters (7) are found. It is assumed that the specific properties of the follicular fluid serum are contributed by other still unkown substances that are important for cell growth, differentiation, and maturation that are contained in the fluid and the serum respectively.

The culture medium containing the cell suspension is incubated under conditions that are useful for growing stem cells, preferably in a CO₂-incubator at a temperature of 30 to 40°C, preferably about 37°C, and in the presence of CO₂, for example 5 or 6 % CO₂. Cells are usually cultured for about 30 days.

These pluripotent stem cells having been obtained from follicular fluid are one of the main aspects of the present invention. They can be grown or can be used in non-differentiated form or can be used to be differentiated into several types of cells.

Moreover, a method for isolating and enriching pluripotent stem cells from the follicular fluid is provided according to the present invention. This method comprises that either cells obtained from OSE or from follicular fluid are isolated by centrifugation, the cell pellet obtained by centrifugation is resuspended in culture medium, the cell suspension is centrifuged with a density gradient, the cloudy layer of cells is recovered after centrifugation and is diluted with culture medium and used for cultivation and/or differentiation.

Using this method results in pluripotent stem cells, which are positive for markers of embryonic stem cells. They express transcript markers Oct-4, SOX-2 and NANOG, as revealed by RT-PCR and are positive for antigen SSEA-4 as revealed by flow cytometry. Moreover, cells that are positive for c-kit, a marker for early germinal cells, can also be obtained, as revealed by immunohistochemistry.

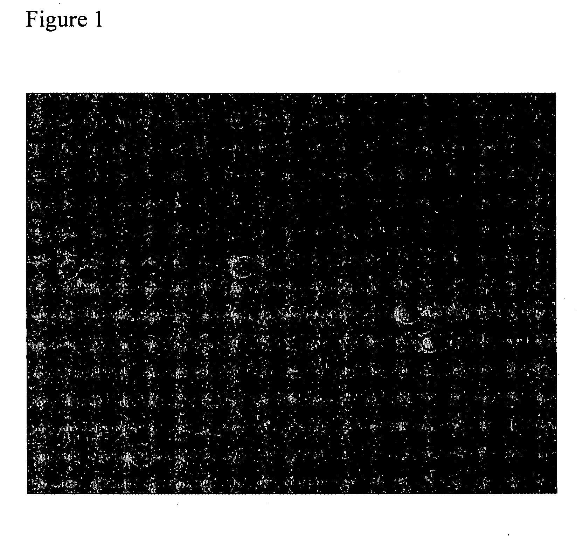

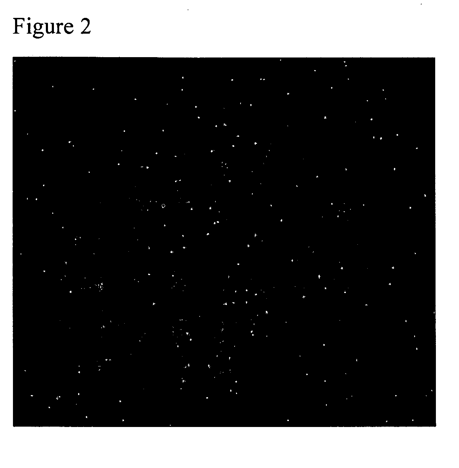

Pluripotent stem cells of the present invention isolated from follicular fluid can be seen in Figures 1 and 2.

The above mentioned method preferably is carried out by subjecting non-coagulated follicular fluid to a first centrifugation step to aggregate cells, and by suspending the aggregated cells in a culture medium. The centrifugation is preferably carried out under conditions that are usual for the aggregation of cells. The centrifugation can be done for a time period of about 5 to 20 min, preferably 7 to 12 min and with 1000 to 5000 rpm, preferably 2000 to 3000 rpm as it is well known to the skilled artisan. Preferably, the centrifugation is performed at about 37°C.

The culture medium used for resuspension of the pellet obtained by centrifugation can be any culture medium that is known for the cultivation of human cells, for example Medium 199:MCDB105, and preferably DMEM/F-12. Additives like hormones, buffering agents, antibiotics and so on can be added as is well known to the skilled artisan. In a preferred embodiment, the culture medium is enriched with up to 30 %, preferably 5 to 25% by volume of the serum of follicular fluid to improve culturing of the cells. Preferably the serum has been heat-inactivated.

The second centrifugation step uses a density gradient for separation of the stem cells. Density gradient products for this purpose are well known in the art. For example products marketed under the name "PureSperm" can be used.

The conditions for the second centrifugation with the density gradient are selected such that the cells can be recovered and are not damaged. It has been found that conditions like using PureSerm for the density gradient centrifugation, such as 100%/40%, with 800 to 1600 rpm for a time period of 5 to 60 min are convenient and yield a fraction visible as a cloudy layer of cells between both concentrations, which can be recovered.

The cloudy layer recovered is diluted in culture medium. This medium can be any medium useful for cell culture and is preferably the same medium as used for resuspension. The best results are obtained when the culture medium contains up to 30 %, preferably 5 to 25% of serum of follicular fluid.

The pluripotent stem cells of the present invention can be grown, can be differentiated or can be frozen and stored until use.

The stem cells of the present invention can be used for differentiation by continuing cultivation under conditions that favor differentiation.

Therefore, a further object of the present invention is a method for differentiating stem cells and for culturing different cell types.

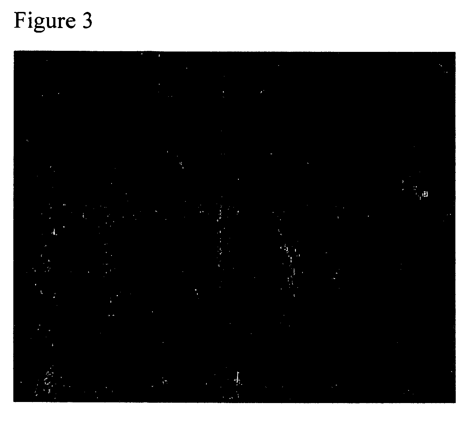

It has been found that different cell types can be obtained from the pluripotent stem cells of the present invention that have been isolated from follicular fluid if the cells are cultured under conditions allowing differentiation. If cells obtained as above are cultured in a medium containing up to 30 %, preferably 5 to 25% of the serum of a follicular fluid, and if attachment of the cells is allowed, fibroblasts are obtained after 2 days of culture as can be seen in Figure 3.

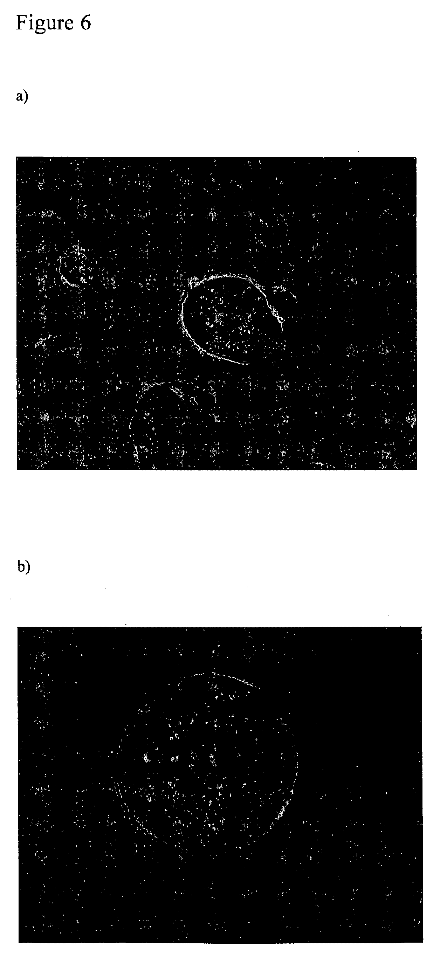

After 4 days of culture oocyte-like cells are found which are confirmed by Oct-4 expression after m-RNA isolation, and c-kit, Oct-4, VASA, DAZL and SCP3 positivity after immunohistostaining. Thus, if it is intended to grow and isolate oocytes, the pluripotent stem cells of the present invention are grown in a medium allowing differentiation for at least 4 days. Oocytes obtained by this method can be seen in Figures 6a) to6c).

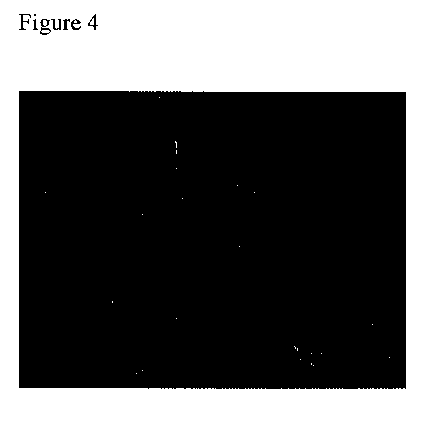

Moreover, it has been found that neurons can be obtained after day about 10 to 14 of culture, which have been confirmed to be positive for antibodies against b3 tubulin (for young neurons) or Map2 (for more mature neurons). Neurons obtained with the stem cells and the method of the present invention are shown in Figure 4.

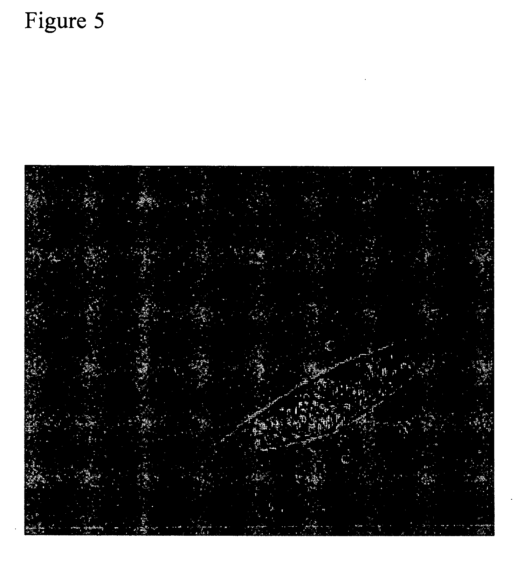

After about 10 to 14 days of culture the stem cells have differentiated to myoblasts that can be recovered as shown above. Myoblasts that have been cultured and differentiated with the method of the present invention are presented in Figure 5.

When the intended type of cells has been differentiated, the cells can be isolated in well-known manner as is known to the skilled artisan. For example the cells can be washed with PBS and released from the dish bottom by addition of a releasing agent, such as 50 % trypsin-EDTA in sterile saline. In this way cells remain viable and can be used for different purposes.

The different cell types obtained after different time periods of culture can be harvested and then be washed by PBS and released from the dish bottom by a mixture of trypsin and EDTA as is known to the skilled artisan. The cells obtained by this method are differentiated cells, are viable and can be used for different purposes such as transplantation or research.

Thus, another object of the present invention is the provision of stem cells for transplantation or for regenerative medicine. The present invention provides the possiblitiy to raise autologous stem cells for transplantation. The invention provides also for the use of heterologous stem cells for regenerative medicine. The present invention allows the use of non-differentiated stem cells and also to first differentiate stem cells to the desireed type of cells and then use the differentiated cells for transplantation and regenerative medicine.

Another important field where the pluripotent stem cells of the present invention can be used is infertility treatment. It has been surprisingly found that the stem cells obtained from follicular fluid can differentiate to become oocytes. Thus, oocytes can be grown from the stem cells and can then be transplanted fertilized after IVF into the uterus of infertile women. Until now those women could become pregnant only with heterologous oocytes or embryos which are rarely available and, like embryonic stem cells, raise ethical problems.

Moreover, with this method it is possible for woman having follicles but cannot yield mature oocytes to grow autologous stem cells out of their immature follicles, differentiate those cells to autologous oocytes that can be fertilized and will result in embryos that can be implanted. This method also allows infertility treatment of infertile women with poor ovarian response to gonadotrophin treatment so called "poor responders" in an in vitro fertilization program. Additional oocytes can be developed from ovarian stem cells in vitro.

Thus, the present invention enables a new approach for the most difficult cases of ovarian infertility treatment.

In a further aspect the present invention provides serum made from follicular fluid as additive to cell cultures for better growth and differentiation of cells in vitro. It has been found that the serum obtained from follicular fluid is very useful for growing cells, particularly stem cells. It can improve growth and differentiation of stem cells. The serum can be obtained from follicular fluid by separating the cells, preferably by centrifugation. In a preferred embodiment the supernatant obtained by centrifugation is inactivated by heat or any other means used in this field.

A preferred embodiment is outlined in the following in more detail. In this embodiment fresh, non-coagulated follicular fluid is centrifuged at 2500 rotations per minute for 10 minutes and the supernatant is filtrated. The filtrate is then heat-inactivated at higher temperature, for example 56°C for 45 minutes in a water bath. The heat-inactivated filtrate (also called serum) can be used as additive for culture media, such as the cell culture medium for ovarian cell culture in a concentration of up to 30 % by volume, preferably 5 to 25 % by volume, optimally at 20 % concentration.

The serum can be used for the cultivation of autologous stem cells, but also as additive to any culture medium used for the cultivation of stem cells or other cells. It was surprisingly found that the serum is very valuable for the cultivation of stem cells as well as differentiated cells. For example, our results show, that in the presence of follicular fluid serum oocyte-like cells can develop in surface epithelium cell stem cell culture in vitro after ovarian biopsate scrapping even in women with premature ovarian failure (POF) with no ovarian function and with no naturally present oocytes, Moreover, it found that the addition of follicular fluid serum improves growth and differentiation of different cell types in vitro, including neurons, because of the very rich chemical composition, especially hormones and growth factors.

The invention wil now be explained in more detail in the following examples.

### Example 1

Set-up of cell culture from the follicular fluid or stem cells isolated from follicular fluid, obtained in IVF program to develop into different cell types in vitro

Fresh, non-coagulated follicular fluid was taken just after oocyte removal for in vitro fertilization. The follicular fluid donor had be tested for the presence of infectious diseases like HIV, hepatitis c and a, and cytomegalovirus (CMV). Follicular fluid was used either fresh or after freeze-thawing. Follicular fluid was used only if the donor was non-infectuous for HIV, hepatitis a and c, and ccytomegalovirus. The whole work was performed under sterile conditions and at a warm (37°C) work place.

### a.) Set-up of cell culture from the follicular fluid:

Ovarian cells were isolated from a non-coagulated fresh or frozen thawed follicular fluid by centrifugation 10 minutes at 2500 rotations per minute (rpm) at about 37°C, After centrifugation the pellet was resuspended in 2 ml of fresh, pre-incubated (in a CO₂-incubator, at least 2 hours) culture medium (DMEM/F-12 (Sigma, USA) with phenol red + NaHCO₃ + antibiotics, pH = 7.4) with added 20 % heat-inactivated serum of the same follicular fluid to obtain cell suspension,. The cell suspension was then added to four-well culture dishes (NUNC) that had been prepared as follows. Each vial was charged with 350 µl of pre-incubated culture medium (DMEM/F-12 with phenol red + NaHCO₃ + antibiotics, pH = 7.4) with added 20 % inactivated serum of the same follicular fluid. 3 to 5 drops of the cell suspension obtained as above was added into each well. The dishes were then placed in a CO₂-incubator at 37°C and 5 % CO₂, The cells were cultured and in the first three days of culture the medium was replaced by fresh medium every day to remove erythrocytes.

### b.) Set-up of cell culture from stem cells isolated from the follicular fluid:

Ovarian cells were isolated from a non-coagulated fresh or frozen thawed follicular fluid by centrifugation 10 minutes at 2500 rotations per minute (rpm). After centrifugation the pellet was resuspended in 2 ml of fresh, pre-incubated culture medium (DMEM/F-12 (Sigma, US) with phenol red + NaHCO₃ + antibiotics, pH = 7.4) with added 20 % inactivated serum of the same follicular fluid to obtain a cell suspension. The cell suspension was centrifuged at 1200 rotations per minute for 30 minutes on a Pure Sperm (NidaCon, Sweden) concentration gradient (2 ml layer of 100 % PureSperm and 2 ml layer of 40 % concentration gradient). After the centrifugation the cell layer (visible as a cloud-tinny layer of cells) between both (100 %/40%) PureSperm fractions was removed and used for cell culture set-up. The cell containing layer was diluted with 1,5 ml of culture medium (DMEM/F-12 with phenol red + NaHCO₃ + antibiotics, pH = 7.4) with added 20 % inactivated serum of the same follicular fluid. To evaluate the tpye of cells that were grown, this suspension of cells was oberved under inverted microscope, two types of until now non-defined round cells were found as can be seen in Figures 1 and 2.

Figure 1: one type were round cells with a bubble-like structure and with approximately 2 to 4 µm diameter, which grew, and intensively proliferated during the culture in vitro. Another type of cells were smaller cells with approximately from 1 to 2 µm diameter, with bubble-like appearance. After m-RNA isolation, the first type of cells are positive for markers of embryonic stem cells like transcript markers Oct-4, SOX-2, and NANOG and antigen SSEA-4. The cells of the first type were found additionally positive for c-kit (marker of early germinal cells) after immunostaining (Figure 2).

Aliquots of 350 µl of this cell suspension were charged into each vial of the two 4-well culture dishes to induce spontaneous differentiation of different cell types in vitro. Moreover, this cell suspension was used to further induce differentiation into a special type of cells (i.e., neurons). The aliquots were incubated in a CO₂-incubator at 37°C and with 5 % CO₂. The stem cells can also be used in non-differentiated form or can be differentiated by different agents.

### Example 2

Spontaneous differentiation of different cell types in a cell culture set-up as in Example 1.)

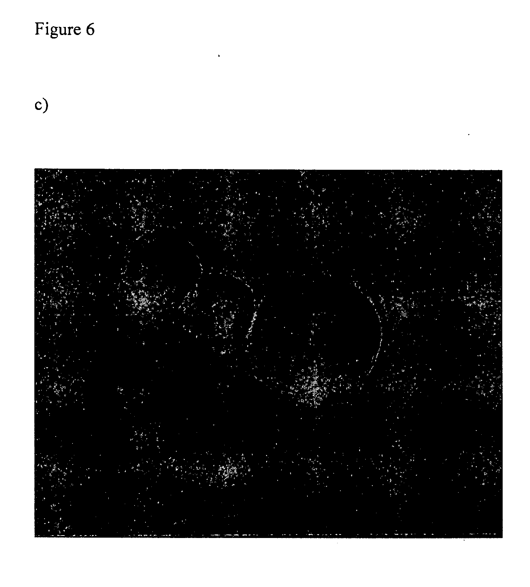

A cell culture was grown for 30 days in a CO₂-incubator at 37°C and 5 % CO₂. Each day samples were observed under an heat-staged inverted microscope at 200 X or 400 X magnification. After day 2 of culture the first attached cells could be observed. Cells attached to the dish bottom and differentiated into different cell types as follows:
fibroblasts after day 2 of culture (than they grow), (see also Figure 3)
granulosa cells after day 4 of culture,
oocyte-like cells after day 4 of culture, (see also Figure 6)
confirmed by Oct-4 expression after m-RNA isolation, and c-kit, Oct-4, VASA, DAZL and SCP3 positivity after immunostaining,
neurons after day 10 to 14 of culture, (see also Figure 4)
are positive for antibodies against b3 tubulin (for young neurons) or Map2 (for more mature neurons),
myoblasts after day 10 to 14 of culture (see also Figure 5).

When cells were differentiated, the cells could be washed by PBS and released from the dish bottom by 50 % trypsin-EDTA in a sterile NaCl. In this way cells viability of the cells is maintained and they can be used for different purposes.

### Example 3

Use of inactivated serum made from follicular fluid, obtained in IVF program as additive to ovarian cell and other cell type cultures for better growth and differentiation of cells in vitro. Fresh or frozen-thawed follicular fluid serum can be used. If frozen, follicular fluid serum is kept at -20°C until use.

Fresh, non-coagulated follicular fluid was centrifuged at 2500 rotations per minute for 10 minutes and supernatant was filtrated. The filtrate was then heat-inactivated at 56°C for 45 minutes in a water bath. After heat-inactivation the filtrate (called serum) of the follicular fluid was used as additive to the cell culture medium for ovarian cell culture (optimally at 20 % concentration) in the same or other women. Follicular fluid of women with a higher number of follicles and oocytes is preferred.

This serum was added also to other cell type cultures (20 % follicular fluid serum solution). Our results show, that in the presence of follicular fluid serum oocyte-like cells can develop in surface epithelium cell stem cell culture in vitro after ovarian epithelium scrapping even in women with premature ovarian failure (POF) with no ovarian function and with no naturally present oocytes. Thus, it could be shown that the addition of follicular fluid serum improves growth and differentiation of different cell types in vitro, including neurons, because of its very rich chemical composition (especially hormones and growth factors).

### References

1 Waldeyer W. Eirstock und Ei. Engelmann, Leipzig, Germany, 1870
2 Kingery HM. Oogenesis in the white mouse. J Morph 1917; 30: 261-315.
3 Allen A. Ovogenesis during sexual maturity. Am J Anat 1923;31:439-481.
4 Evans HM and Swezy O. Ovogenesis and the normal follicular cycle in adult mammalia. Mem Univ Calif 1931;9:119-224.
5 Byskov AG, Addy MJ, Lemmen JG, Andersen CY. Eggs forever ? Differentiation 2005; 73: 438-46.
6 Hutt KJ, Albertini DF. Clinical applications and limitations of current ovarian stem cell research: a review. J Exp Clin Assist Reprod 2006; 27: 3-6.
7 Skaznik-Wikiel M, Tilly JC, Lee HJ, Niikura Y, Kaneko-Tarui T, Johnson J, Tilly JL. Serious doubts over »Eggs forever« ? Differentation 2007; 75: 93-9.
8 Szotek PP, Pieretti-Vanmarcke R, Masiakos PT, Dinulescu DM, Connolly D, Foster R, Dombkowski D, Preffer F, Maclaughlin DT, Donahoe PK. Ovarian cancer side population defines cells with stem cell-like characteristics and Mullerian Inhibiting Substance responsiveness. Proc natl Acad Sci USA 2006; 103(30): 11154-9.
9 Johnson J, Canning J, Kaneko T, Pru JK, Tilly JL. Germline stem cells and follicular renewal in the postnatal mammalian ovary. Nature 2004; 428: 145-50.
10 Johnson J, Bagley J, Skaznik-Wikiel M, Lee HJ, Adams GB, Niikura Y, Tschudy KS, Till JC, Cortes ML, Forkert R, Spitzer T, Iacomini J, Scadden DT, Tilly JL. Oocyte generation in adult mammalian ovaries by putative germ cells in bone marrow and peripheral blood. Cell 2005; 122: 303-15.
11 Wright WE, Piatyszek MA, Rainey WE, Byrd W, Shay JW. Telomerase activity in human germline and embryonic tissues and cells. Dev Genet 1996; 18(2): 173-9.
12 Parrott JA, Kima G, Skinner MK. Expression and Action of Kit Ligand/Stem Cell Factor in Normal Human and Bovine Ovarian Surface Epithelium and Ovarian Cancer. Biology of Reproduction 2000; 62: 1600-9.
13 Bukovsky A, Svetlikova M, Caudle MR. Oogenesis in cultures derived from adult human ovaries. Reprod Biol Endocrinol 2005; 3: 17.
14 Quinn MC, McGregor SB, Stanton JL, Hessian PA, Gillett WR, Green DP. Purification of granulosa cells from human ovarian follicular fluid using granulosa cell aggregates. Reprod Fertil Dev 2006; 18(5): 501-8.
15 Heng BC, Cao T, Bested SM, Tong GQ, Ng SC (2005) "Waste" follicular aspirate from fertility treatment-a potential source of human germline stem cells ? Stem Cells Dev 2005; 14(1): 11-14.
(16) Duijkers IJ, Willemsen WN, Hollanders WN, Hamilton CJ, Thomas CM, Wemer HM. Follicular fluid hormone concentrations after ovarian stimulation using gonadotropin preparations with different FSH/LH ratios. II. Composition of hMG and recombinant FSH. Int J Fertil Womens Med 1997; 42(6)431-5.
(17) Velasquez A, Reyes A, Chargoy J, Rosado A. Amino acid and protein concentrations of human follicular fluid. Fertil Steril 1977; 28(1):96-100.
(18) Bokal EV, Tacer KF, Vrbnjak M, Leposa S, Virant-Klun I, Verdenik I, Rozman D. Follicular sterol composition in gonadotrophin stimulated women with polycystic ovarian syndrome. Moll Cell Endocrinol 2006; 249(1-2): 98-8.
(19) Ulug E, Turan E, Tosun SB, Erden HF, Bahceci M. Comparison of preovulatory follicular concentrations of epidermal frowth factor, insulin-like growth factoe-I, and inhibins A and B in women undergoing assisted conceptions treatment with gonadotropin-releasing hormone (GnRH) agonists and GnRH antagonists. Fertil Steril 2007; 87(4): 995-8.
(20) Eriksen GV, Carlstedt I, Morgelin M, Uldbjerg N, Malmstrom A. Isolation and characterization of proteoglycans from human follicular fluid. Biochem J 1999; 340(3): 613-20.
(21) Jaspard B, Collet X, Barbaras R, Manent J, Vieu C, Parinaud J, Chap H, Perret B. Biochemical characterization of pre-beta 1 high density lipoprotein from human ovarian follicular fluid: evidence for the presence of a lipid core. Biochemistry 1996; 35(5): 1352-7.
(22) Pahuja SL, Kim AH, Lee G, Hochberg RB. Origin of estradiol fatty acid esters in human ovarian follicular fluid. Biol Reprod 1995; 52(3): 625-30.
(23) Smitz J, Picton HM, Platteau P, Rutherford A, Cortvrindt A, Clyde J, Nogueira D, Devroey P, Lyby K, Grondahl C. Principal findings from a multicenter trial investigating the safety of follicular fluid meiosis-activating sterol for in vitro maturation of human cumulus-enclosed oocytes. Fertil Steril 2007; 87(4): 949-46.

## Claims

1. Pluripotent stem cells obtained from follicular fluid.

2. Method for obtaining pluripotent stem cells comprising isolating cells from follicular fluid and culturing the isolated cells in a culture medium.

3. Method according to claim 2, wherein the culture medium contains up to 30% by volume of serum of follicular fluid.

4. Method according to claim 2 or 3, wherein the culture medium contains 5 to 25% by volume of follicular fluid serum.

5. Method according to one of claims 2 to 4, wherein the follicular fluid used as source for the cells and the serum are both from the same donor.

6. Method according to one of claims 2 to 4, wherein the follicular fluid for obtaining cells and the serum are from different donors.

7. Method according to one of claims 2 to 6, wherein follicular fluid is centrifuged to aggregate stem cells, the cell pellet obtained after centrifugation is resuspended in culture medium, the cell suspension is added to a culture medium and is grown for at least 1 day wherein the medium is replaced every day.

8. A method for isolating pluripotent stem cells from follicular fluid wherein follicular fluid is centrifuged to aggregate stem cells, the cell pellet obtained after centrifugation is resuspended in culture medium, the cell suspension is centrifuged using a density gradient, the cell containing layer is recovered and diluted with culture medium and the diluted cell suspension is grown in culture medium thereby producing pluripotent stem cells.

9. Method according to claim 8 wherein the culture medium contains up to 30% by volume of follicular fluid serum.

10. Method for differentiation of stem cells into different cell types wherein a cell suspension containing stem cells is grown in the presence of follicular fluid serum.

11. Method for differentiation of stem cells into different cell types wherein a cell suspension as obtained with a method of one of claims 2 to 9 is cultured under conditions allowing differentiation and differentiated cells are recovered and released.

12. The method of claim 11 wherein the stem cells are cultivated in the presence of follicular fluid serum.

13. Use of non-differentiated stem cells of claim 1 from for transplantation or research.

14. Use of differentiated cells obtained from pluripotent stem cells of claim 1 for transplantation.

15. Use of oocyte-like cells obtained from pluripotent stem cells of claim 1 for the treatment of infertility.

16. Use of one of claim 13 to claim 15 wherein the pluripotent stem cells have been differentiated in culture medium containing serum of follicular fluid.

17. Culture medium for growing and differentiating stem cells containing up to 30 % by volume serum of follicular fluid.

18. The culture medium of claim 16 containing 5 to 25 % by volume of serum of follicular fluid.

19. Culture medium for growing cells containing up to 30 % by volume serum of follicular fluid.

20. The culture medium of claim 14 or 15 containing additionally buffering agents, antibiotics, growth enhancing agents and has a pH of 6 to 8.
